# NEW EUROPEAN PATENT SPECIFICATION

(11) **EP 0 130 775 B2**
(45) Date of publication and mention of the opposition decision: **30.06.1993**
(45) Mention of the grant of the patent: 14.10.1987
(21) Application number: 84304309.2
(22) Date of filing: 26.06.1984
(51) Int. Cl.: C12N 1/38, A23C 19/032, A23C 9/123

(54) **An improved process for making dairy products**
Verfahren zur Herstellung von Molkereiprodukten
Procédé de préparation de produits laitiers

(30) Priority: 27.06.1983 US 507928; 16.09.1983 US 532673
(43) Date of publication of application: 09.01.1985
(73) Proprietor: Bily, Robert Raymond, San Jose California 95127 (US)
(72) Inventor: Bily, Robert Raymond, San Jose California 95127 (US)
(74) Representative: Baillie, Iain Cameron

(56) References cited:
- DE-C- 697 440
- US-A- 3 278 313
- US-A- 4 110 476
- CHEMICAL ABSTRACTS, vol. 49, 1955, column 16054g,h, Columbus, Ohio, USA; R.H. DEIBEL et al.: "The minute streptococci: further studies on their nutritional requirements and growth characteristics on blood agar"
- JOURNAL OF DAIRY SCIENCE, vol. 57, no. 5, 1974, page 582; C.A. SPECKMAN et al.: "Direct inoculation of milk with lyophilized starter concentrates for yogurt production"
- CHEMICAL ABSTRACTS, vol. 85, no. 3, 19th July 1976, page 519, no. 19233e, Columbus, Ohio, USA; R.K. BAISYA et al.: "Effect of addition of different chemical additives to milk upon the quality of freeze dried curd (dahi) powder"
- Mair-Waldburg, Handbuch der Käse, Volkswirtschaftlicher Verlag GmbH, Kempten (1974), page 235
- Fermented Fresh Milk Products, Vol. 1, "Yoghurt", by J.Lj. Rasic, (1978), page 203
- H. Mulder and P. Walstra, "The milk fat globuline", Commonwealth Agricultural Bureaux Farnham Royal, Bucks, GB and Centre for Agricultural Publishing and Documentation Wageningen, NL, (1974), pp. 27 and 30
- Dutch law on Agricultural Products quality, Regulations on Milk and Dairy Products, Section 22
- "Zuivelzicht", 72(1980), 32-33, page 679
- "Specification of dairy products used as raw materials for recombining", Neth. Milk Dairy J., 42 (1988)
- International Dairy Federation Bulletin, Document 116 (1979)

## Description

This invention relates to a process for making dairy products.

Any improvement in a dairy medium that results in faster acid production, and/or increased activity of an acid producing bacterial culture, and/or increased storage viability is of commercial value to the cultured dairy products industry, since it speeds up production and reduces the amount of starter culture necessary. Decreased dairy medium requirements reduce the overall energy consumption since less energy is needed for sterilization and maintenance of fermentation temperatures. Also improved culture activity and viability during refrigerated storage also allows use of cultures over longer periods of time, necessitating less frequent batch production.

There are a number of steps in the production of cultured dairy foods. Conventionally when making cheese, for instance, milk is placed in a vat and heated to an appropriate temperature. A starter culture is added to ripen the milk and after an appropriate time, rennet extract or milk-coagulating catalyst is mixed in to initiate the coagulation process (unless the milk is setwith starter culture only). The amount of milk, starter culture and rennet extract or coagulating catalyst and of many other ingredients which are added is closely controlled. The resulting mixture is allowed to remain quiet until a curd forms. The curd is then cut and cooked prior to draining or dipping, knitting, salting and pressing. Such starter cultures are also used in other acidic, cultured dairy products such as buttermilk, sour cream and yoguhrt.

According to the present invention there is provided a process for making a lactic food product which comprises incubating acid-producing bacteria in a sterile carrier medium to which at least 0.01 % lecithin by weight has been added either prior to or after sterilisation, said lecithin being additional to any lecithin which may be present naturally in said carrier medium due to biosynt hesis, to produce an active, viable starter culture, adding said starter culture to a lactic medium and fermenting the resulting mixture.

It has been found that the addition of lecithin enhances bacterial growth and/or viability of acid, especially lactic acid, producing bacteria and provides a buffering effect in certain cultured dairy media increasing their long term storage viability.

The process of this invention may be used with various dairy or other special carrier media suitable for the growth or suspension of acid producing bacteria e.g. lactic starter cultures. Since other acids are produced in the fermentation of dairy media, all acid producing bacteria produced in cultured dairy media can be employed. Examples of lactic starter cultures include bulk starter cultures, frozen cultures, concentrated bacterial cultures and frozen concentrated bulk starters. A "dairy medium" or "lactic medium" is any medium capable of supporting acid producing bacteria that has as, at least one of its ingredients, a dairy product such as whey, milk, cream or any milk derivative such as whey protein, casein, lactose or other milk components that can be used as acid producing dairy media. A "special carrier medium" is a broader term including, additionally, any medium useful as a preincubation, resuspending or acid producing medium that is harmless to lactic starter cultures and that can be used to solubilize, mix and carry lecithin in suspension with said cultures prior to inoculation of a lactic medium with said cultures; examples include the lactic media described and liquids such as water that are harmless to acid producing bacteria and that can be mixed with lecithin.

When preparing the acid producing dairy medium, lecithin may be added directly to the milk, whey, cream or other dairy product being used in, for example, the production of bulk starter. Furthermore, when "direct-vat-set" cultures are used as starters such as those that have been concentrated and/or frozen for direct addition to dairy products, or when lecithin is added to bulk starter medium that has already been inoculated, a carrier medium with lecithin mixed therein such as uncultured lactic medium, or other medium that can serve as a harmless preincubation; resuspending or acid producing carrier medium suitable for saturating starter cultures with lecithin can be made; these "direct-vat-set" cultures or bulk starter cultures may first be added to the premix carrier medium to saturate said bacteria with lecithin before their addition to the dairy food to be fermented. The object is to surround the bacteria with an ample supply of lecithin before addition to the dairy product to be fermented. This action enhances the activity of the bacteria when they are added to the dairy product to be fermented in the same way bacterial activity is enhanced in bulk starters which are previously produced with lecithin incorporated in the starter media prior to inoculation. Any lecithin derived from animal or vegetable sources, which is normally a natural mixture of phospholipids or phosphatides derived from soybeans can be used. Liquid lecithin can be used, but powdered lecithin that is substantially oil free is easier to mix in aqueous solutions. Lecithin is best added to the chosen medium by prolonged high shear agitation and heat. The presence of these two factors ensures total solubility of the lecithin in the medium. The temperature of the medium should generally be at least 140°F (60°C) at the time the lecithin is solubilized. Generally, lecithin is added in amounts from .01 to 10% by weight of the medium.

The method in which the lecithin is added to the medium should be carefully controlled. For example, if the lecithin is added to a dairy media before it is inoculated, it is best to add the lecithin directly to it while employing high shear agitation and high heat as in the production of bulk starter media. If the dairy medium has previously been inoculated, it is best not to vigorously agitate it when adding the lecithin water inoculation to avoid bacterial shear and oxygen toxicity. In this case, the lecithin should desirably be added to a quantity of a premix carrier medium, which when vigorously mixed together under high heat can be added to the starter culture with gentle agitation; the resulting mixture is then allowed to stand for, say, 1 to 10 minutes so that saturation of the bacteria with lecithin can occur before adding the entire mixture to the dairy food product medium to be fermented.

Different methods of mixing under high shear agitation can be used. The lecithin can be mixed vigorously with the dairy medium in a regular culture tank before inoculation and processed in the same way as any bulk starter culture media; or if a premix carrier medium is to be made first, the lecithin can be mixed with a premix carrier medium with a weight ratio of about 1:10 to 1:30 in a high shear mixer such as a Viking mixer, or the lecithin can be added to the premix carrier medium via a funnel and reciprocating high speed pump that recirculates the mixture vigorously back through the pump until solubility of the lecithin is achieved.

Other ingredients may be present, for example phosphate salts, dextrose, pancreatin and yeast, in dairy media such as bulk starter media, like in milk or whey or both.

The following Examples further illustrate the present invention.

### Example 1

Lowfat milk (2% fat and 10% solids-not-fat) was autoclaved at 250°F (121°C) for 10 minutes and then cooled to 120°F (49°C). Metarin K powdered soybean lecithin (Lucas Meyer, Inc.) was weighed into a portion of the warmed milk to produce an 18% w/v solution, which was then blended at high speed on a Waring Commercial Blenderfor 10 minutes. The blended lecithin solution was then diluted with three parts of warmed sterile lowfat milk to obtain a final lecithin concentration of 4.5%. A control medium consisting of sterile lowfat milk without lecithin added was also prepared. Initial pH of each medium was 6.60.

Six Streptococcus cremoris starter culture strains (DPL Culture Service, Inc., San Francisco) used in commercial dairy operations were inoculated into the 4.5% lecithin medium and the control milk. Two of the strains were obtained as freeze-dried DPL Bulk Set concentrated starter powders and were inoculated in the proportion recommended for commercial bulk starter preparation. The remaining four strains were inoculated at a 1 % level from fresh milk cultures that had been ripened at 73°F (23°C) for 18 hours. This level simulated handling of factory grown intermediate starter cultures.

Each inoculated starter medium was incubated at 73°F (23°C) for 16 hours as in commercial practice. The pH of each culture was then measured; that of cultures grown employing 4.5% lecithin was found to be consistently higher than that of the controls.

Each ripened culture was inoculated at 5% into pasteurized nonfat milk containing 9.0% solids-not-fat. Samples were incubated at 88°F (31°C) to simulate commercial short-set cottage cheese-making conditions. The time required for production of a curd with cutting firmness is shown by Table 1, Quicker set times were observed for those obtained using lecithin for at least five out of the six strains (denoted as A to F).

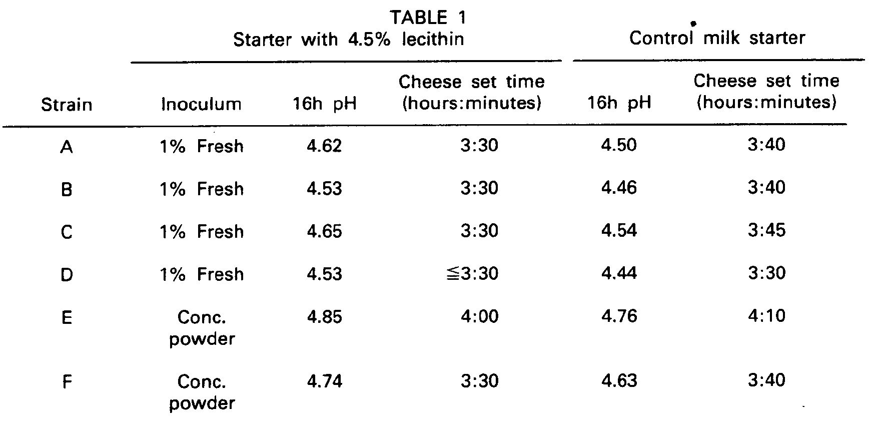

### Example 2

Control and 4.5% lecithin starter media were prepared as in Example 1. The medium was Ellikers Agar from Difco. Three Streptococcus cremoris strains were inoculated into each medium at 1%. Each inoculated starter culture was incubated at 73°F for 17 hours to permit maximum acid development and cell growth, each culture was then checked for pH and cell density.

Table 2 shows that the pH of cultures grown in the medium of the invention was consistently higher than that of the controls. The additional data presented in Table 2 shows that the same density of cells was achieved in both cases. Accordingly the higher pH is due not to retarded cell growth resulting in less acid production but to a superior buffering effect, this being generally recognized as beneficial to culture activity and stability.

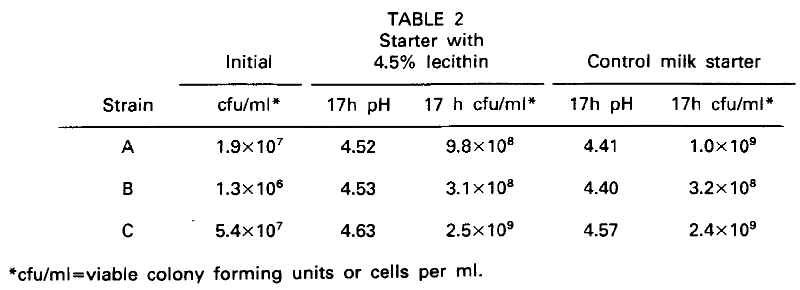

### Example 3

Control and 4.5% lecithin milk media were prepared as in Example 1 and adjusted to 88°F prior to inoculation with two Streptococcus cremoris strains that had been ripened in lowfat milk at 73°F for 18 hours at 2%. The cultures were incubated at 88°F, the optimum growth temperature for Streptococcus cremoris.

The growth of each culture was followed throughout incubation by monitoring acid development. Cell densities were checked at time of inoculation, in the middle of the fermentation, and the ripened culture.

Table 3 shows that, despite the buffering effects of lecithin, the pH of both strains, enhanced in the lecithin medium, fell more rapidly than in the controls, particularly in the second half of the fermentations. Cell densities were very similar at the 2.5 hour point, but both cultures made with lecithin showed superior cell densities in the ripened culture.

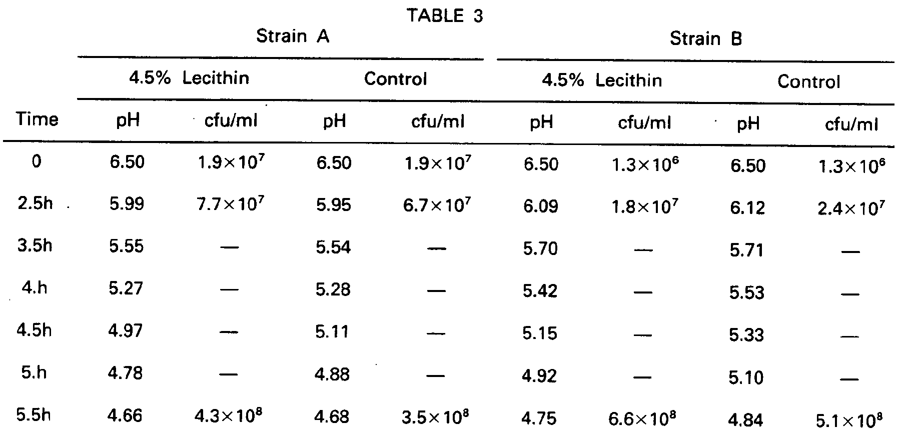

### Example 4

Example 3 was repeated using a premix carrier medium containing 10% lecithin (Lec. in tables) by weight of the premix weighing 200 pounds (91 kg), employing a wider range of strains, and monitoring growth by pH development only in the food product mil. The premix carrier medium with lecithin was prepared by adding Metarin K powdered lecithin to lowfat milk at 140°F (60°C) and blending the warm mixture at high speed for 5 minutes. The resulting medium was tempered to 88°F and admixed with 5% of freshly ripened milk culture of each culture strain individually. This lecithin-culture-carrier medium premix was allowed to stand for about 5 minutes and added at 10% to the food product milk. Six commercial strains of Streptococcus lactis and cremoris (denoted by G to L), representing a range of activity, temperature, and bacteriophage sensitivity properties, were each incubated separately in said product milk at 88°F. Similar strains not enhanced with lecithin were added to food product milk individually to serve as controls.

Table 4 shows that the rate of culture development, as represented by acid production, was more rapid when lecithin was used for all six strains studied.

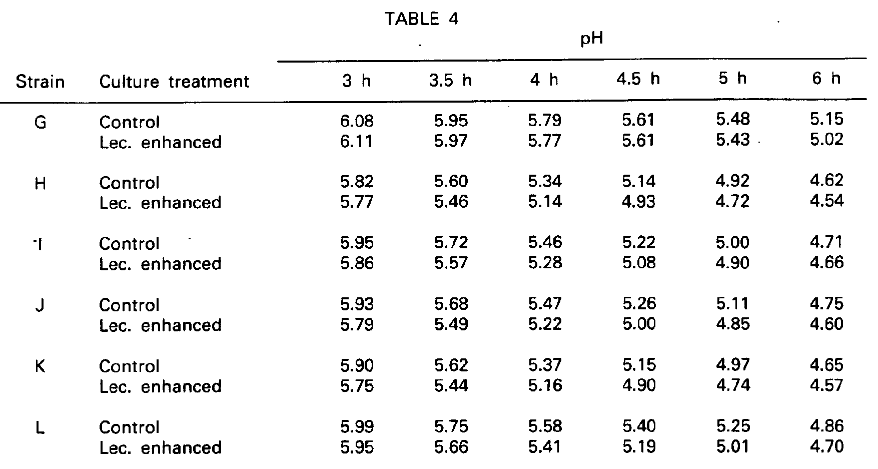

### Example 5

The effect of the lecithin-culture-carrier medium premix prepared in Example 4 on the activity of Lactobacillus acidophilus, a thermophilic lactic acid producing bacterium, was tested at two incubation temperatures. The premix was tempered to either 100°F (38°C) or 113°F (45°C) and admixed with 5% of a fresh milk culture of Lactobacillus acidophilus. The mixture was allowed to stand for 10 minutes and then added to the food product milk which was to be fermented.

Growth of the cultures was monitored by following pH development over 6.5 hours. Table 5 shows that acid development at both 100° and 113°F was increased as compared to the control.

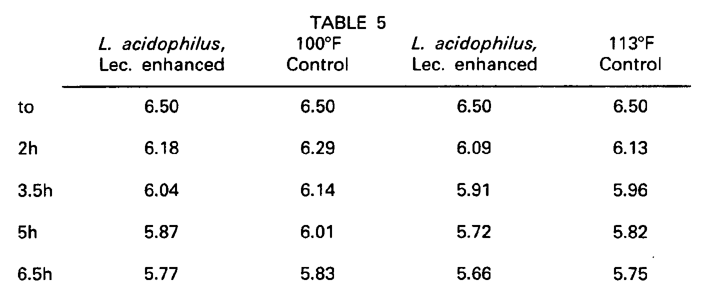

### Example 6

The effect of 1.0% lecithin milk medium in Streptococcus cremoris culture activity, after storage at 40°F (4°C) was examined. 1% Lecithin by weight was added to milk as shown beforehand and compared to control milkwithout lecithin. Three strains of Streptococcus cremoris ripened in lowfat milk at 73°F (23°C) for 18 hours were inoculated at 1 % into the media tempered to 73°F. Cultures were incubated at 73°F for 18 hours and chilled to 40°F.

Activity of each culture was tested on days 0 (freshly ripened), 1, 4 and 7 by inoculating at 5% into pasteurized nonfat milk containing 9.0% solids-not-fat, incubating at 88°F (31°C) and checking the pH after 3.5 hours. Table 6 shows that all three strains grown in 1.0% lecithin medium show a slight to significant improvement in acid-producing activity on Day 4 and a substantial improvement in activity on Day 7 as compared to their activity in control milk medium.

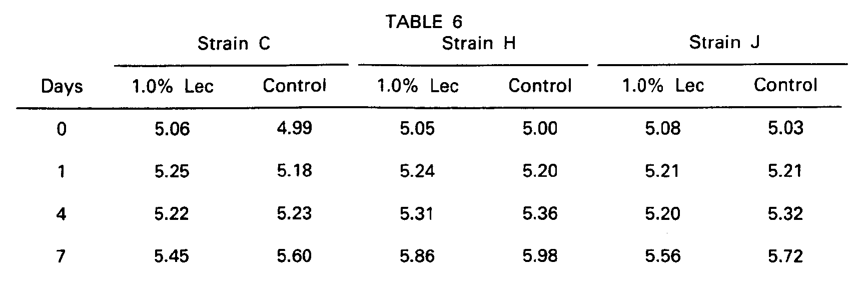

### Example 7

The effect of very low levels of lecithin on culture activity in five strains of Streptococcus cremoris and lactis was examined. Test milk medium was prepared by adding either 0.05% or 0.10% by weight of Metarin K powdered lecithin to low fat milk at 140°F (60°C), blending the warm mixture at high speed for 5 minutes, and then autoclaving at 250°F for 10 minutes. The medium with and without lecithin was cooled to 73°F and inoculated with 1 % freshly ripened milk culture of each of the strains to be tested. Cultures were incubated at 73°F (23°C) for 18 hours and then tested for pH and activity.

Activity of each ripened culture was checked by inoculating at 2% into 9.0% nonfat milk, incubating at 88°F (31 °C), and checking the pH after 3.5 hours. Table 7 shows that pH development for all five cultures was generally superior for 0.05% and 0.10% lecithin milk grown cells as compared to the control. Though the exact levels at which lecithin benefits various representative strains may differ, a measurable improvement in culture activity was observed in all strains examined at either 0.05% or 0.10% lecithin.

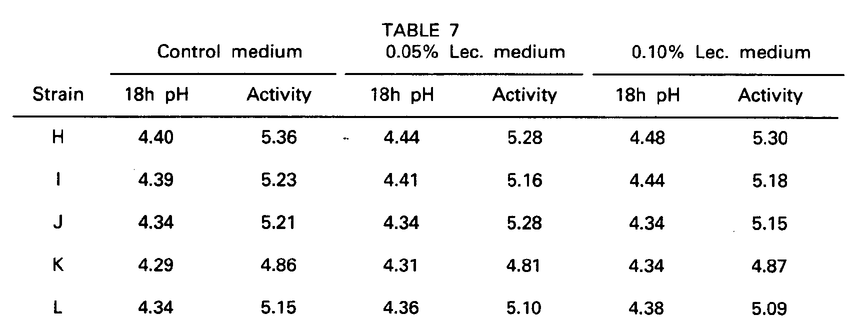

### Example 8

The effect of presoaking bacterial cultures in a solution ofwaterand lecithin on culture activity after addition to product milk is examined using a strain of Streptococcus cremoris. In this Example, water acted as the special carrier medium to solubilize the lecithin and resuspend and preincubate the bacterial cultures added thereto. This special medium was prepared by adding 1.25% Metarin K powdered lecithin by weight to distilled water at 145°F (63°C) and employing high shear agitation for2 minutes. The mixture was then sterilized at 12 pounds for 10 minutes. A sterilised control was prepared in the same way but without lecithin.

Both media were admixed with 20% of fresh mother culture and preincubated for 10 minutes (first test) and 18 hours (second test) at 70°F (21 °C). Activity of each culture was checked by inoculating at 2% into 10% nonfat milk, incubating at 88°F (31°C), and checking the pH at 4 hours and 5 hours. A further control was included by adding 2% of fresh mother culture directly into 10% nonfat milk under identical methods but without lecithin or preincubation.

Table 8 shows that pH reduction, i.e. acid development, for cultures preincubated in the special carrier media made with lecithin, was superior to the controls. Also the lecithin enhanced cultures created firmer sets more quickly in the test milks.

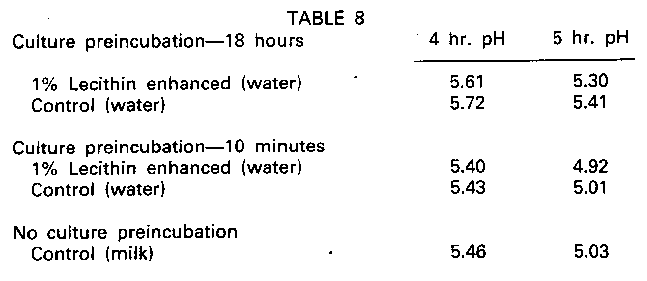

## Claims

1. A process for making a lactic food product which comprises incubating acid-producing bacteria in a sterile carrier medium to which at least 0.01% lecithin by weight has been added either prior to or after sterilisation, said lecithin being additional to any lecithin which may be present naturally in said carrier medium due to biosynthesis, to produce an active, viable starter culture, adding said starter culture to a lactic medium and fermenting the resulting mixture

2. A process according to claim 1 wherein said carrier medium is a lactic medium.

3. A process according to claim 1 or 2, wherein lecithin is added to the said medium under conditions of high shear and at a temperature of about 140°F (60°C).

4. A process according to any one of claims 1 to 3, wherein the acid producing bacteria are lactic acid producing bacteria.

5. A process according to any one of the preceding claims, wherein the carrier medium contains 0.01 to 10% lecithin by weight.

6. A process accordingly to any one of the preceding claims for making cheese, buttermilk, sour cream or yoghurt.

7. A process according to any one of the preceding claims wherein the medium to be incubated is obtained by adding powdered lecithin and the acid producing bacteria to the carrier medium.

8. A process for producing an active, viable lactic food starter culture characterised by incubating acid-producing bacteria in an appropriate sterile carrier medium to which at least 0.01% lecithin by weight has been added either prior to or after sterilisation, said lecithin being additional to any lecithin which may be present naturally in said carrier medium due to biosynthesis.

9. A process for producing a culture medium and bacterial culture characterised by preparing said culture or culture medium by combining acid-producing bacteria with a sterile culture medium and by combining at least 0.01 % lecithin by weight of the culture medium with said culture medium either prior to or after sterilisation of said culture medium, subsequently incubating the mixture of culture medium, acid-producing bacteria and lecithin to produce an active, viable culture.

10. A process for preparing a bacterial culture characterised by combining acid-producing bacteria with a sterile culture medium and incubating the mixture to produce a culture and by combining at least 0.01 % lecithin by weight of the culture with said culture, subsequently incubating the mixture of culture and lecithin to produce an active viable culture.

## Patentansprüche

1. Verfahren zum Herstellen eßbarer Milchprodukte durch Züchten säure-produzierender Bakterien in einem sterilen, vor- oder nach der Sterilisation mit wenigstens 0,01 Gew.% Lezithin versetzten Trägermedium zur Herstellung einer aktiven lebensfähigen Starterkultur, wobei das genannte Lezithin zusätzlich zu aufgrund von Biosynthesen naturgemäß im Trägermedium befindlichem Lezithin vorhanden ist, Hinzufügen dieser Starterkultur zu einem Medium auf Milchbasis und Fermentation der erhaltenen Mischung.

2. Verfahren nach Anspruch 1, bei welchem das Trägermedium ein Medium auf Milchbasis ist.

3. Verfahren nach Anspruch 1 oder2, bei welchem dem Trägermedium Lezithin unter hoher Scherbeanspruchung und bei einer Temperatur von etwa 60°C zugesetzt wird.

4. Verfahren nach einem der Ansprüche 1 bis 3, bei welchem die säure-produzierenden Bakterien milchsäure-produzierende Bakterien sind.

5. Verfahren nach einem der vorausgehenden Ansprüche, bei welchem das Trägermedium 0,01 Gew.% bis 10 Gew.% Lezithin enthält.

6. Verfahren nach einem der vorausgehenden Ansprüche zur Herstellung von Käse, Buttermilch, Sauerrahm oder Joghurt.

7. Verfahren nach einem der vorausgehenden Ansprüche, bei welchem das Inkubationsmedium durch Zusatz von pulverisiertem Lezithin und säureproduzierenden Bakterien zum Trägermedium erhalten wird.

8. Verfahren zur Herstellung einer aktiven, lebensfähigen Starterkultur für eßbare Milchprodukte,
gekennzeichnet durch
Züchten säure-produzierender Bakterien in einem geeigneten sterilen Trägermedium, welchem entweder vor oder nach der Sterilisation wenigstens 0,01 Gew.% Lezithin zugefügt wurden, wobei das Lezithin zusätzlich zu dem aufgrund von Biosynthesen naturgemäß im Trägermedium befindlichen Lezithin vorhanden ist.

9. Verfahren zum Herstellen eines Kulturmediums und einer Bakterienkultur,
gekennzeichnet durch
Bereiten des Kulturmediums bzw. der Bakterienkultur durch Kombination säure-produzierender Bakterien mit einem sterilen Kulturmedium und durch Zusatz entweder vor oder nach der Sterilisation von wenigstens 0,01 Gew.% Lezithin bezogen auf das Gewicht des Kulturmediums zu diesem Kulturmedium, sowie nachfolgendem Bebrüten der Mischung aus Kulturmedium, säure-produzierenden Bakterien und Lezithin zur Herstellung einer aktiven lebensfähigen Kultur.

10. Verfahren zum Herstellen einer Bakterienkultur,
gekennzeichnet durch
Kombination säure-produzierender Bakterien mit einem sterilen Kulturmedium und Bebrüten der Mischung zur Herstellung einer Kultur und Kombination von wenigstens 0,01 Gew.% Lezithin bezogen auf das Gewicht der Kultur mit dieser Kultur, sowie nachfolgendem Bebrüten der Mischung aus Kultur und Lezithin zum Herstellen einer aktiven lebensfähigen Kultur.

## Revendications

1. Procédé de préparation d'un produit alimentaire lactique, qui consiste à faire incuber des bactéries productrices d'acide dans un milieu porteur stérile dans lequel on a ajouté au moins 0,01 % en poids de lécithine, soit avant, soit après la stérilisation, ladite lécithine venant en plus de toute lécithine qui serait naturellement présente dans le milieu porteur par suite de biosynthèse, de façon à produire une culture d'amorçage viable et active, à ajouter cette culture d'amorçage à un milieu lactique et à faire fermenter le mélange obtenu.

2. Procédé selon le revendication 1, dans lequel ledit milieu porteur est un milieu lactique.

3. Procédé selon la revendication 1 ou 2, dans lequel on ajoute la lécithine audit milieu sous des conditions de cisaillement élevé et à une température d'environ 140°F (60°C).

4. Procédé selon l'une quelconque des revendications 1 à 3, dans lequel les bactéries productrices d'acide sont des bactéries productrices d'acide lactique.

5. Procédé selon l'une quelconque des revendications précédentes, dans lequel le milieu porteur contient de 0,01 à 10 % de lécithine en poids.

6. Procédé selon l'une quelconque des revendications précédentes pour préparer du fromage, du babeurre, de la crème acide ou du yaourt.

7. Procédé selon l'une quelconque des revendications précédentes, dans lequel on obtient le milieu à faire incuber en ajoutant de la lécithine en poudre et les bactéries productrices d'acide au milieu porteur.

8. Procédé de préparation d'une culture d'amorçage, viable et active, pour produit alimentaire lactique, caractérisé en ce qu'on fait incuber des bactéries productrices d'acide dans un milieu porteur stérile approprié contenant au moins 0,01 % de lécithine en poids, ladite lécithine venant en plus de toute lécithine qui serait naturellement présente dans le milieu porteur par suite de biosynthèse.

9. Procédé de préparation d'un milieu à culture et d'une culture bactérienne, caractérisé en ce qu'on prépare ladite culture ou ledit milieu de culture en combinant des bactéries productrices d'acide avec un milieu de culture stérile et en combinant audit milieu de culture au moins 0,01 % de lécithine en poids par rapport au milieu de culture, soit avant, soit après stérilisation dudit milieu de culture, en faisant ensuite incuber le mélange du milieu de culture, des bactéries productrices d'acide et de lécithine de façon à produire une culture viable et active.

10. Procédé de préparation d'une culture bactérienne, caractérisé en ce que l'on combine des bactéries productrices d'acide avec un milieu de culture stérile et on fait incuber le mélange de façon à produire une culture et en ce que l'on combine à ladite culture au moins 0,01 % de lécithine en poids par rapport à la culture, puis on fait ensuite incuber le mélange de culture et de lécithine de façon à produire une culture viable et active.
